# EUROPEAN PATENT SPECIFICATION

(11) **EP 2 864 333 B1**
(45) Date of publication and mention of the grant of the patent: **19.07.2017**
(21) Application number: 12770289.2
(22) Date of filing: 25.06.2012
(51) Int. Cl.: C07D 491/052, A61K 31/407, A61P 31/14

(54) **ETODOLAC DERIVATIVES AS HCV NS5B POLYMERASE INHIBITORS**
ETODOLAC-DERIVATE ALS HCV-NS5B-POLYMERASEHEMMER
DÉRIVÉS D'ÉTODOLAC UTILISÉS EN TANT QU'INHIBITEURS DE LA POLYMÉRASE NS5B DU VHC

(43) Date of publication of application: 29.04.2015
(73) Proprietor: Kucukguzel, S. Guniz, 34718 Istanbul (TR)
(72) Inventor: SUZGUN, Pelin, 34381 Istanbul (TR); ARORA, Payal, Newark, New Jersey 07103 (US); BASU, Amartya, Newark, New Jersey 07103 (US); KAUSHIK-BASU, Neerja, Newark, New Jersey 07103 (US)
(74) Representative: Mutlu, Aydin
(86) International application number: PCT/TR2012/000108
(87) International publication number: WO 2014/003693

(56) References cited:
- WO-A1-00/77006
- WO-A1-03/099275
- GOPLSAMY A ET AL: "Discovery of Pyrano[3,4-b]indoles as Potent and Selective HCV NS5B Polymerase Inhibitors", JOURNAL OF MEDICINAL CHEMISTRY, AMERICAN CHEMICAL SOCIETY, US, vol. 47, 26 December 2004 (2004-12-26), pages 6603-6608, XP002361050, ISSN: 0022-2623, DOI: 10.1021/JM0401255
- Q.-B. Song ET AL: "Crystal structure of (1,8-diethyl-1,3,4,9-tetrahydro-pyrano[3,4 -b]indol- 1-yl)acetic acid (2-hydroxy-benzylidene)hydrazide, C24H27N3O3", ZEITSCHRIFT FUER KRISTALLOGRAPHIE - NEW CRYSTAL STRUCTURES, vol. 221, no. 3, 1 January 2006 (2006-01-01), XP055224704, DE ISSN: 1433-7266, DOI: 10.1524/ncrs.2006.0076

## Description

### FIELD OF THE INVENTION

The present invention relates to thiosemicarbazide and 1,2,4-triazole derivatives from etodolac hydrazide which can be useful for the inhibition of hepatitis C virus NS5B RNA dependent RNA polymerase activity.

### BACKGROUND OF THE INVENTION

Hepatitis C virus is mentioned with hepatocellular cancer. Hepatitis C virus (HCV) is a single strand RNA virus, identified in 1989, from the *Flaviviridae* strain. It is an important human pathogen threating the world population with chronic hepatitis, cirrhosis hepatocellular carcinoma and chronic liver diseases. Recently, in HCV therapy with ribavirin combined with interferon-α, important side effects are observed and there is no preventive inoculation in this virus which forms a serious extent of danger for human health. In its HCV genome, there are structural and non-structural proteins. Its structural proteins (C, E1 ve E2) are in the polyprotein amino ending. Its non-structural proteins are in the carboxyl ending of polyproteine (NS2 to NS5B) and they are liberalized on the virus side.

HCV NS5B protein is a 66 kDa phosphoprotein and it is dominantly localised around the nucleus and it codes the RNA linked RNA polymerase (RdRp). RNA linked RNA polymerase (RdRp) coded with NS5B protein belonging HCV is the central enzyme in the replication of HCV genome and it presents an ideal target for drugs having small molecular structure. Recently, the interest for targetting the allosteric linking site of NS5B placed on the lower effective areas in the head part which is far from the polimerase active site has been increased. Therefore, a number of work related to the new hepatitis C virus replication inhibitors have been carried out.

Studies on 1,3,4,9-tetrahydropyrano[3,4-*b*]indole structure which forms the main skeleton of etodolac active substance, attracted attention as HCV NS5B polimerase enzyme inhibitors. Gopalsamy et al.; designed and synthesised a new serial of HCV NS5B RdRp inhibitors in the structure of 1,2,3,4- tetrahydro-cyclopenta[b]indole (Gopalsamy et al., "Discovery of Pyrano[3,4-b]indoles as Potent and Selective HCV NS5B Polymerase Inhibitors", J. Med. Chem., 2004, 47 (26), pp 6603-6608). They established that [(R)-5-cyano-8-methyl-1-propyl-1,3,4,9-tetrahydropyrano[3,4-*b*]indole -1-yl]acetic acid derivatives exhibited the largest activity with IC₅₀values of 0.3-1.4 µM for the 90% of genotypes against NS5B enzyme derived from HCV genotype. In a previous study published in 2010, La Porte et. al. reported that pyrano[3,4-d]indol derivatives inhibited HCV NS5B enzyme with an IC50 value of 0.002 µM.

Kaushik-Basu et al. evaluated the inhibitor effect of eighteen compound from the synthesised 4-thiazolidine derivatives against HCV NS5B RdRp. They obtained IC₅₀ values of 45-75 microM following the in vitro HCV NS5B RdRp inhibition analysis (Kaushik-Basu N, Bopda-Waffo A, Talele TT, Basu A, Chen Y, Küçükgüzel 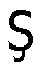 G. 4-Thiazolidinones: A novel class of hepatitis C virus NS5B polymerase inhibitors. Front Biosci, 2008, 13, 3857-3868).

Ivanov et al. established that 2,3,4- and 3,4,5-trihydroxybenzaldehyde hydrazones have higher HCV RdRp activites than 3,4-dihydroxy derivatives (Ivanov, A.V., Kozlov, M.V., Kuzyakin, A. O., Kostyuk, D.A., Tunitskaya, V. L., Kochetkov, S. N., "New Non-nucleoside Inhibitors of Hepatitis C Virus RNA-Dependent RNA Polymerase", Biochemistry (Moscow), Vol. 69, No. 7, 2004, pp. 782-788. Translated from Biokhimiya, Vol. 69, No. 7, 2004, pp. 959-967). The same researchers in 2006 reported that the cyanoacetylhydrazone and carboxyacetylhydrazone derivative from pyrogallol derivative hydrazones increased the inhibition compared to the acetylhydrazone derivative (Kozlov, M.V., Polyakov, A. V., Ivanov, A.V., Filippova, S. E., Kuzyakin, A. O., Tunitskaya, V. L., Kochetkov, S. N., "Hepatitis C Virus RNADependent RNA Polymerase: Study on the Inhibition Mechanism by Pyrogallol Derivatives", 2006, published in Biokhimiya, 2006, Vol. 71, No. 9, pp. 1253-1259).

However, there is no screening study reported for 1,2,4-triazole-3-thione and semicarbazide structures for the HCV NS5B efficiencies in the literature.

In the light of this information, the etodolac derivatives comprising 1,3,4,9-tetrahydropyrano[3,4-b]indole structure were synthesized. It was investigated how this inhibitor effect is affected by compounds with different structures, previously reported for NS5B polymerase inhibitor effect. For this purpose new compounds have been synthesized by starting from etodolac.

### DETAILED DESCRIPTION OF THE INVENTION

In this invention, the hepatitis C virus NS5B polymerase enzyme inhibition of compounds in the structure of hydrazide-hydrazone, 4-thiazolidone, thiosemicarbazide and 1,2,4-triazole synthesised by etodolac were studied.

When the hepatitis C virus NS5B polymerase enzyme inhibition effects of etodolac derivatives were screened, it was found that the effect of 4-thiazolidones had lower effects against Wedelolacton Standard; while the other derivatives being thiosemicarbazide and 1,2,4-triazole-3-thiones were more effective with their IC50 values. Among these, the most active thiosemicarbazide compounds were **SGK 224** (1-[2-(1,8-diethyl-1,3,4,9-tetrahydropyrano[3,4-b]indol-1-yl)acetyl]-4-methyl thiosemicarbazide) and **SGK 229** (1-[2-(1,8-Diethyl-1,3,4,9-tetrahydropyrano[3,4-b]indole-1-yl)acetyl]-4-allyl thiosemicarbazide) with IC₅₀ values of 18.7 µM and 16.8 µM, respectively. The most active 1,2,4-triazole compound was **SGK238** (5-[(1,8-diethyl-1,3,4,9-tetrahydropyrano[3,4-b]indol-1-yl)methyl]-4-methyl-2,4-dihydro-3H-1,2,4-triazole-3-thione) with IC₅₀ value of 14.8 µM.

In the present invention, for studing hepatitis C virus NS5B polymerase enzyme inhibition, preferably, methyl (1,8-diethyl-1,3,4,9-tetrahydropirano[3,4-*b*]indole-1-yl) acetate [**SGK 196**], 2-(1,8-diethyl-1,3,4,9-tetrahydropyrano[3,4-*b*]indole-1-yl)acetohydrazide [**SGK 197**], 2-(1,8-diethyl-1,3,4,9-tetrahydropyrano[3,4-*b*]indole-1-yl) acetic acid [(5-nitro-2-furyl/substituted phenyl)methylene] hydrazides [**SGK 198-210**], 3-(2-(1,8-diethyl-1,3,4,9-tetrahydropyrano[3,4-*b*]indole-1-yl)acetylhydrazono)-2-alkyl/aryl-4-thiazolidinones **[SGK 211-214, SGK 216-221, SGK 223],** 1-[2-(1,8-diethyl-1,3,4,9-tetrahydropyrano[3,4-b] indol-1-yl)acetyl]-4-alkyl/aryl thiosemicarbazides [**SGK 224-230, SGK 313**] and 5-[(1,8-diethyl-1,3,4,9-tetrahydropyrano[3,4-b]indol-1-yl)methyl]-4-substituted-2,4-dihydro-3H-1,2,4-triazole-3-thione [**SGK 238-244, SGK 315**] compounds were selected, which were synthesized by starting from (*R*,*S*)-2-[1,8-diethyl-1,3,4-tetrahydrapyrano[3,4-*b*]indole-1-yl]acetic acid (etodolac).

The compounds 1-[2-(1,8-diethyl-1,3,4,9-tetrahydropyrano[3,4-b] indol-1-yl)acetyl]-4-alkyl/aryl thiosemicarbazides [**SGK 224-230, SGK 313**] and 5-[(1,8-diethyl-1,3,4,9-tetrahydropyrano[3,4-b]indol-1-yl)methyl]-4-substituted-2,4-dihydro-3H-1,2,4-triazole-3-thione [**SGK 238-244, SGK 315**] have been found to be advantageous, and therefore the invention pertains to these compounds exclusively.

### Effect on HCV NS5B polymerase enzyme inhibition

### Material and Methods

### Purification of recombinant replicase proteins

Plasmid pThNS5BCdelta21 was transformed in *Escherichia coli* DH5alpha and used for purification of HCV NS5BCdelta21 as previously described. The plasmid pet24b-SARS-CoV-nsp12 was transformed in E. coli BL21 STAR DE3 (Invitrogen) to express a Cterminally His-tagged SARS-CoV RdRp. The cells were cultured in LB medium supplemented with kanamycin (30 microgram/mL) at 37°C until the culture density (A600) reached 0.6. After induction of expression with 0.2 mM isopropyl-beta-D-1-thiogalactopyranoside (IPTG), the cells were grown for 16 h at 18°C. Cells were harvested and the His-tagged nsp12 was purified by Ni-NTA affinity chromatography as described for HCV NS5B. Identity of the purified SARS-CoV-nsp12-His protein was confirmed by western blotting employing the His-probe (H3) mouse monoclonal primary antibody and goat anti-mouse horseradish peroxidase conjugated secondary antibody. Membrane-bound antibodies were detected with the ECL enhanced chemenhanced chemiluminescence kit.

### 4RNA-dependent RNA polymerase (RdRp) assay

The RdRp activity of NS5B was evaluated by the standard primer-dependent elongation reactions employing poly rA/U12 template-primers (TP). Reaction mixtures containing 20 mM Tris-HCl (pH 7.0), 100 mM NaCl, 100 mM Na-glutamate, 0.5 mM DTT, 0.01% BSA, 0.01% Tween-20, 5% glycerol, 20 U/ml of RNasin, 0.5 microM of poly rA/U12, 25 microM UTP, 2-5 microCi [alpha-32P]UTP, 500 ng of NS5BCdelta21 and 0.5 mM MnCl2 with or without inhibitors in a total volume of 25 microliters were incubated for 1 h at 30°C. Reactions were quenched by the addition of ice cold 5% (v/v) trichloroacetic acid (TCA) containing 0.5 mM pyrophosphate (PPi), the denatured polymeric RNA products were transferred to GF-B filters and the amount of radioactive UMP incorporated into RNA products was quantified on a liquid scintillation counter (Packard). The concentrations of the compounds inhibiting 50% of NS5B RdRp activity (IC50) were calculated from the inhibition curves as a function of inhibitor concentration. Nsp12 activity was similarly evaluated except that incubations were carried out at 25°C for optimal enzymatic activity.

Incorporation of UMP on poly rA/U12 template primer by NS5BCdelta21 at varying compound concentrations was visualized qualitatively by gel analysis of reaction products essentially as described above for the HCV RdRp assay except that the elongation time was reduced to 20 min. Reactions were stopped by the addition of 25 mM EDTA-0.5% SDS, subjected to phenolchloroform extraction and ethanol precipitation and the recovered RNA products were dissolved in formamide gel loading buffer and resolved on a denaturing 12% polyacrylamide gel containing 7 M urea. The extent and pattern of synthesis was visualized by phosphorimaging (Molecular Dynamics). The primer-independent de novo initiation activity of NS5B in the presence of compound 6 was reconstituted on HCV plus-strand 5'-UTR RNA template under conditions as previously described. Synthesis of the HCV plus-strand 5'-UTR run-off RNA transcript was carried out in vitro employing the HCV 5' UTR PCR product carrying the T7 RNA polymerase promoter at its 5'terminus as template and the T7 RNA polymerase kit in accordance with the manufacturer's procedure.

The ability of the compounds to inhibit HCV NS5B RdRp activity was investigated in vitro by polyrA-U12 extension assay as described in experimental section by Kaushik-Basu N, et al.. The compounds **SGK 224-230, 313** and **SGK 238- 244, 315** were reconstituted in DMSO as 50 mM stocks, and serially diluted in DMSO to obtain working stocks.

Preliminary screening was carried out at 100 µM to identify a wider range of compounds. Percentage inhibition of HCV NS5B RdRp activity was determined at 0.1 mM concentration of the indicated compound and represents an average of at least two independent measurements in duplicate. NS5B RdRp activity in the absence of the inhibitor was taken as 100 percent after subtraction of residual background activity. The concentration of DMSO in all reactions was kept constant at 5%. The IC₅₀ values of compounds exhibiting ≥50% inhibition at 0.1 mM concentration were determined from dose-response curves using 8-10 concentrations of each compound in duplicate in two independent experiments. Curves were fitted to data points using nonlinear regression analysis and IC₅₀ values were interpolated from the resulting curves using GraphPad Prism 3.03 software.

The invention which is set forth here is essentially to study for the Hepatitis-C NS5B polymerase enzyme inhibition efficiencies.

Etodolac, the parent molecule included in this investigation for comparison with its derivatives, exhibited the lowest activity against NS5B of ∼10%. Previously reported as an inhibitor of NS5B, and the IC 50 value of 36.0 mM Wedelolakton, used as internal reference standard.

The etodolac derivatives exhibited varying degree of HCV NS5B polymerase inhibition ranging from ∼6.6% to 86.0% at 100 µM concentration (Tables 1-2). Among these, the most active thiosemicarbazide compounds were **SGK 224** and **SGK 229** with IC₅₀ values of 18.7 µM and 16.8 µM, respectively. The most active 1,2,4-triazole compound was **SGK 238** with IC5₀ value of 14.8 µM (Table 3). In the present study Wedelolactone is used as a reference standard. When compared with reference standard Wedelolactone, 2-(1,8-Diethyl-1,3,4,9-tetrahydropyrano[3,4-*b*]indole-1-yl) acetic acid [(3-fluorophenyl) methylene] hydrazide **SGK 202** compound, bearing 3-fluorophenyl substituent, in the structure of hydrazone was seen to have an equal efficiacy; Since IC₅₀ values of **SGK 224, SGK 227, SGK 229, SGK 238-241** compounds are lower than Wedelolactone, these compounds were thought to be effective. In these series, **SGK 238** compound, bearing methyl substituent and having a IC₅₀ value of 14.8 µM, in the structure of 1,2,4-triazole-3-thione was established to be the most effective compound. These are new type of compounds which have not yet reported as HCV NS5B polymerase inhibiting compounds in the literature

**Table 1: Activities of Etodolac Derivatives for Hepatitis C NS5B Polymerase Enzyme Inhibition**

| **Compd.** | **Anti-NS5B Activity (% Inhibition, 200 µM)** | **Anti-NS5B Activity (% Inhibition, 100 µM)** |
|---|---|---|
| **SGK 196** | 13.3±1.2 | 6.4±0.9 |
| **SGK 197** | 19.0±4.4 | 11.4±0.2 |

| **Etodolac Hydrazide-hydrazones** | | |
|---|---|---|
| **SGK 198** | 12.1±4.1 | 6.4±1.3 |
| **SGK 209** | 63.0±5.5 | 53.7±0.5 |
| **SGK 199** | 23.8±1.8 | 16.5±1.8 |
| **SGK 200** | 36.5±6.3 | 30.3±0.2 |
| **SGK 201** | 16.5±3.7 | 6.7±1.0 |
| **SGK 202** | 66.4±5.8 | 61.6±0.4 |
| **SGK 203** | 6.7±4.6 | 3.4±0.4 |
| **SGK 204** | 11.3±4.5 | 9.2±0.9 |
| **SGK 205** | 14.1±6.7 | 8.4±1.4 |
| **SGK 206** | 13.2±3.9 | 7.1±0.4 |
| **SGK 207** | 6.6±3.1 | 3.6±1.1 |
| **SGK 208** | 25.6±6.0 | 20.0±0.1 |
| **SGK 210** | 52.1±4.3 | 45.3±1.1 |

| **Etodolac Thiosemicarbazides** | | |
|---|---|---|
| **SGK 224** | 77.5±1.3 | 76.2±0.5 |
| **SGK 225** | 59.6±7.3 | 49.9±0.3 |
| **SGK 226** | 38.9±5.8 | 34.6±1.1 |
| **SGK 227** | 73.3±2.4 | 68.7±0.9 |
| **SGK 229** | 81.6±0.8 | 78.6±2.1 |
| **SGK 228** | 29.1±5.8 | 23.4±1.8 |
| **SGK 230** | 45.2±2.3 | 38.9±0.9 |
| **SGK 313** | 55.1±3.0 | 47.4±0.9 |

**Table 2: Activities of Etodolac Derivatives for Hepatitis C NS5B Polymerase Enzyme Inhibition**

| **Compound** | **Anti-NS5B Activity (% Inhibition, 200 µM)** | **Anti-NS5B Activity (% Inhibition, 100 µM)** |
|---|---|---|
| **Etodolac 1,2,4-Triazoles** | | |
| **SGK 238** | 86.0±0.6 | 78.9±0.7 |
| **SGK 239** | 76.9±0.4 | 74.6±1.1 |
| **SGK 240** | 74.4±3.8 | 69.0±0.1 |
| **SGK 241** | 75.4±2.4 | 72.3±0.4 |
| **SGK 243** | 65.8±1.5 | 58.2±0. |
| **SGK 242** | 15.5±3.9 | 3.78 |
| **SGK 244** | 56.8±1.9 | 49.5±0.1 |
| **SGK 315** | -* | 45.6 |

| **Etodolac 4-Thiazolidinones** | | |
|---|---|---|
| **SGK 211** | 37.0±2.4 | 1.80 |
| **SGK 212** | 25.6±1.3 | 1.01 |
| **SGK 213** | 33.3±2.9 | 6.66 |
| **SGK 214** | 36.4±0.3 | 13.22 |
| **SGK 216** | 45.9±4.9 | 40.3±0.4 |
| **SGK 217** | 25.3±5.9 | ineffective |
| **SGK 218** | -* | 13.1 |
| **SGK 219** | -* | ineffective |
| **SGK 220** | -* | 43.2 |
| **SGK 221** | -* | 45.2 |
| **SGK 223** | -* | 26.3 |

### (Anti-HCV Activity (%Inhibition, 100 µM Etodolac: 7.4)

**Table 3: IC₅₀ values for Etodolac Derivatives**

| **Compound** | **Anti-NS5B Activity (% Inhibition, 100 µM)** | **IC₅₀ (µM)** |
|---|---|---|
| **SGK 209** | 53.7±0.5 | 58.6±1.4 |
| **SGK 202** | 61.6±0.4 | 35.9±1.4 |
| **SGK 224** | 76.2±0.5 | 18.7±1.2 |
| **SGK 227** | 68.7±0.9 | 29.2±1.4 |
| **SGK 229** | 78.6±2.1 | 16.8±1.2 |
| **SGK 238** | 78.9±0.7 | 14.8±1.2 |
| **SGK239** | 74.6±1.1 | 21.6±1.3 |
| **SGK 240** | 69.0±0.1 | 26.0±1.3 |
| **SGK 241** | 72.3±0.4 | 25.4±1.3 |
| **SGK 243** | 58.2±0. | 40.3±1.2 |
| **Wedelolacton** | | 36.0 |

## Claims

1. A compound having one of the formulas:
1-[2-(1,8-diethyl-1,3,4,9-tetrahydropyrano[3,4-b] indol-1-yl)acetyl]-4-alkyl/aryl thiosemicarbazides, and
5-[(1,8-diethyl-1,3,4,9-tetrahydropyrano[3,4-b]indol-1-yl)methyl]-4-substituted-2,4-dihydro-3H-1,2,4-triazole-3-thione.

2. The compound according to claim 1 which is 5-[(1,8-Diethyl-1,3,4,9-tetrahydropyrano[3,4-b]indol-1-yl)methyl]-4-methyl-2,4-dihydro-3H-1,2,4-triazole-3-thione having IC₅₀ value of 14.8 µM for the hepatitis C virus NS5B polymerase enzyme inhibition.

3. The compound according to claim 1 which is 1-[2-(1,8-diethyl-1,3,4,9 tetrahydro pyrano[3,4-b]indol-1-yl)acetyl]-4-methyl thiosemicarbazide or 1-[2-(1,8-Diethyl-1,3,4,9-tetrahydropyrano[3,4-b]indole-1-yl)acetyl]-4-allyl thiosemicarbazide having IC₅₀ values of 18.7 µM and 16.8 µM, respectively, for the hepatitis C virus NS5B polymerase enzyme inhibition.

## Patentansprüche

1. Verbindung mit einer der Formeln:
1-[2-(1,8-Diethyl-1,3,4,9-tetrahydropyrano[3,4-b]indol-1-yl)acetyl]-4-(alkyl/aryl)thiosemicarbazide; und
5-[(1,8-Diethyl-1,3,4,9-tetrahydropyrano[3,4-b]indol-1-yl)methyl]-4-(substituiert)-2,4-dihydro-3H-1,2,4-triazol-3-thion.

2. Verbindung gemäß Anspruch 1, bei der es sich um 5-[(1,8-Diethyl-1,3,4,9-tetrahydropyrano[3,4-b]indol-1-yl)methyl]-4-methyl-2,4-dihydro-3H-1,2,4-triazol-3-thion mit einem IC₅₀-Wert von 14,8 µM für die Hemmung des Enzyms NS5B-Polymerase des Hepatitis-C-Virus handelt.

3. Verbindung gemäß Anspruch 1, bei der es sich um 1-[2-(1,8-Diethyl-1,3,4,9-tetrahydropyrano[3,4-b]indol-1-yl)acetyl]-4-methylthiosemicarbazid mit einem IC₅₀-Wert von 18,7 µM oder 1-[2-(1,8-Diethyl-1,3,4,9-tetrahydropyrano[3,4-b]indol-1-yl)acetyl]-4-allylthiosemicarbazid mit einem IC₅₀-Wert von 16,8 µM für die Hemmung des Enzyms NS5B-Polymerase des Hepatitis-C-Virus handelt.

## Revendications

1. Composé ayant l'une des formules :
1-[2-(1,8-diéthyl-1,3,4,9-tétrahydropyrano[3,4-b]indol-1-yl)acétyl]-4-alkyl/aryl-thiosemicarbazides, ou
5-[(1,8-diéthyl-1,3,4,9-tétrahydropyrano[3,4-b]indol-1-yl)méthyl]-4-substitué-2,4-dihydro-3H-1,2,4-triazole-3-thione.

2. Composé selon la revendication 1, qui est la 5-[(1,8-Diéthyl-1,3,4,9-tétrahydropyrano[3,4-b]indol-1-yl)méthyl]-4-méthyl-2,4-dihydro-3H-1,2,4-triazole-3-thione ayant une CI₅₀ de 14,8 µM pour l'inhibition de la polymérase NS5B du virus de l'hépatite C.

3. Composé selon la revendication 1, qui est le 1-[2-(1,8-diéthyl-1,3,4,9-tétrahydropyrano[3,4-b]indol-1-yl)acétyl]-4-méthy-thiosemicarbazide ou le 1-[2-(1,8-Diéthyl-1,3,4,9-tétrahydropyrano[3,4-b]indole-1-yl)acétyl]-4-allyl-thiosemicarbazide ayant respectivement des CI₅₀ de 18,7 µM et 16,8 µM pour l'inhibition de la polymérase NS5B du virus de l'hépatite C.
